# EUROPEAN PATENT SPECIFICATION

(11) **EP 2 249 816 B1**
(45) Date of publication and mention of the grant of the patent: **20.05.2015**
(21) Application number: 09720545.4
(22) Date of filing: 10.03.2009
(51) Int. Cl.: A61K 9/50, A61K 31/663

(54) **FORMULATION AND METHOD FOR THE PREVENTION AND TREATMENT OF BONE METASTASES OR OTHER BONE DISEASES**
FORMULIERUNG UND VERFAHREN ZUR VERHINDERUNG UND BEHANDLUNG VON KNOCHENMETASTASEN ODER ANDEREN KNOCHENERKRANKUNGEN
FORMULATION ET PROCÉDÉ POUR LA PRÉVENTION ET LE TRAITEMENT DES MÉTASTASE OSSEUSES ET D AUTRES MALADIES DES OS

(30) Priority: 10.03.2008 FR 0851521
(43) Date of publication of application: 17.11.2010
(73) Proprietor: Erytech Pharma, 69008 Lyon (FR)
(72) Inventor: BOURGEAUX, Vanessa, F-69007 Lyon (FR); GODFRIN, Yann, F-69004 Lyon (FR)
(74) Representative: Colombet, Alain André
(86) International application number: PCT/EP2009/052792
(87) International publication number: WO 2009/112493

(56) References cited:
- CN-A- 1 771 972
- DATABASE CA [Online] CHEMICAL ABSTRACTS SERVICE, COLUMBUS, OHIO, US; HIRAOKA, KOJI ET AL: "Metastasis inhibitor containing liposomal bisphosphonate-based agents" XP002501431 retrieved from STN Database accession no. 2008:160529 & WO 2008/016172 A1 (KURUME UNIVERSITY, JAPAN) 7 February 2008 (2008-02-07)
- ROSSI LUIGIA ET AL: "Macrophage depletion induced by clodronate-loaded erythrocytes." JOURNAL OF DRUG TARGETING FEB 2005, vol. 13, no. 2, February 2005 (2005-02), pages 99-111, XP008097945 ISSN: 1061-186X
- ROSSI LUIGIA ET AL: "Prolonged islet allograft survival in diabetic mice upon macrophage depletion by clodronate-loaded erythrocytes." TRANSPLANTATION 27 FEB 2008, vol. 85, no. 4, 27 February 2008 (2008-02-27), pages 648-650, XP008097941 ISSN: 0041-1337

## Description

The invention relates to formulations and medicaments for the treatment of bone metastases and other bone diseases. It also relates to methods of prevention and of treatment.

Bisphosphonates are synthetic analogues of pyrophosphate (structure P-O-P) in which the central oxygen atom is replaced with a carbon atom. Their chemical structure can be represented by the following formula:

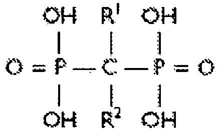

Bisphosphonates can be placed in two categories.

The first category comprises the "first-generation" compounds which do not contain a nitrogen atom in their side chains R¹ and R². This category comprises, in particular, etidronate, clodronate and tiludronate.

The secondary category comprises the "second-generation" and "third-generation" compounds which contain one or more nitrogen atoms in one of their side chains R¹ or R². Those of the second generation comprise an aliphatic side chain bearing a nitrogen atom or a terminal NH₂ group. Mention should be made of pamidronate, alendronate, ibandronate and neridronate. Those of the third generation bear a heterocyclic nucleus containing a nitrogen atom. Mention should be made of risedronate and zoledronate (imidazole nucleus).

This classification as first, second and third generation is entirely known to those skilled in the art. By way of illustration, mention will be made of T. Yuasa et al., Current Medical Chemistry 2007, 14: 2126-2135 and Selvaggi et al., Crit. Rev. Oncol. Hematol. 2005, 56(3): 365-378; V. Stresing et al., Cancer Letters 2007, 257: 16-35; R. Graham G. Russell et al., Ann. N.Y. Acad. Sci. 2007, 1117: 209-257.

Bone metastases are common in the case of advanced cancer. They are most common in multiple myelomas, breast cancer and prostate cancer, but are also present in the case of melanoma and in the case of bladder cancer, lung cancer and kidney cancer.

Bisphosphonates have become essential in the therapeutic treatment of patients suffering from bone cancer. Clodronate, pamidronate, zoledronate and ibandronate are thus used.

Bisphosphonates can, however, be toxic at high doses. They can also be subject to considerable renal elimination. For example, for zoledronate, this elimination by the kidneys generates a renal toxicity such that the doses used clinically in humans must be carefully supervised and may find themselves below the effective doses for the treatment of bone metastases. Thus, the clinical doses of zoledronate are 10 to 40 times lower than the effective doses determined in animals (J. Green, Oncologist 2004, 9: 3-13).

L. Rossi et al. (Journal of Drug Targeting, February 2005, 13(2): 99-111) describes the encapsulation of clodronate in erythrocytes, and the use thereof for the depletion of macrophages. They also describe treatment of the erythrocytes with ZnCl₂ and BS3, in the presence of ethanolamine and of bovine serum albumin. The authors demonstrate a depletion of spleen macrophages in mice. The study neither concerns nor envisages the use of erythrocytes as a bisphosphonate vector in bone applications.

K. Hiraoka et al. (WO2008016172) describes the use of clodronate encapsulated in liposomes as an agent for inhibiting bone metastasis and/or muscle metastasis. CN1771972 also discloses liposomes and clodronate for use in the treatment of bone metsatases. L. Rossi et al. (Transplantation, 2008, 85, 4:648-650) describes that clodronate encapsulated in erythrocytes prolongs islet allograft survival in diabetic mice.

The objective of the invention is therefore to propose a solution for delivering bisphosphonates at the level of the bone marrow, thereby limiting renal elimination and avoiding the problems of toxicity which currently limit the efficacy of these active ingredients, according to claim 1.

An objective of the invention is also to propose such a solution which makes it possible to increase the bioavailability of bisphosphonates at the level of the bone marrow.

An objective of the invention is also to propose such a solution which makes it possible to decrease the amount of bisphosphonate administered for a given treatment and compared with the free form.

An objective of the invention is also to propose such a solution which makes it possible to increase the administrable dose, without experiencing the limiting problems of toxicity of the free form.

A subject of the invention is thus a suspension of erythrocytes encapsulating a bisphosphonate, for use as a medicament for targeting the bone marrow and for bringing the bisphosphonate to this bone marrow, limiting or even eliminating any risk of toxicity, in particular renal toxicity.

A subject of the invention is also a suspension of erythrocytes encapsulating a bisphosphonate, for use as a carrier for bringing a bisphosphonate into the bone marrow, in particular for the treatment or prevention of bone diseases such as bone metastases.

By definition, the term "bisphosphonate" encompasses all the bisphosphonates, and the salts and derivatives thereof, and in particular the first-, second- and third-generation bisphosphonates and the salts and derivatives thereof. This term therefore encompasses diphosphonates, biphosphonic acids and diphosphonic acids.

Nonlimiting examples of bisphosphonates include: etidronate, clodronate, tiludronate, pamidronate, in particular the disodium form, alendronate, incadronate, ibandronate, neridronate, risedronate and zoledronate.

Preferably, the invention uses bisphosphonates containing at least one nitrogen atom, for example second-generation and third-generation bisphosphonates. In particular selected are: pamidronate, alendronate, ibandronate and neridronate for the second generation. Those of the third generation bear a heterocyclic nucleus containing a nitrogen atom. Mention should be made of risedronate and zoledronate (imidazole nucleus).

The invention relates in particular to a suspension of erythrocytes encapsulating a bisphosphonate, for use as a medicament for the prevention and treatment of bone and bone marrow pathologies for which bisphosphonates may be indicated. It makes it possible to provide optimized doses for the treatment of the pathology targeted, in so far as the treatment protocol is no longer limited by the toxicity associated with the free form of the bisphosphonate.

Thus, the use may relate to the prevention or treatment of bone metastases, of malignant hypercalcaemia, of Paget's disease and of osteoporosis.

The invention is more particularly a suspension of erythrocytes encapsulating a bisphosphonate, for use as a medicament for the prevention and treatment of bone metastases.

The invention also relates to a medicament containing a suspension of erythrocytes encapsulating a bisphosphonate, for use for the prevention and treatment of these bone or bone marrow diseases, and in particular of bone metastases.

The invention also relates to the use of a suspension of erythrocytes encapsulating a bisphosphonate, for the preparation of a medicament for use in the prevention and treatment of these bone or bone marrow diseases, and in particular of bone metastases.

The invention also relates to a suspension of erythrocytes encapsulating a bisphosphonate, for use as a carrier for bringing a bisphosphonate into the bone marrow, for the treatment or prevention of bone metastases.

Preferably, the encapsulation of the bisphosphonate is carried out by means of a procedure referred to as lysis-resealing.

The bisphosphonate is generally prepared in a buffered solution, for example PBS, at pH 7.2 to 7.6, preferably at 7.4.

According to one characteristic of this procedure, the lysis of the erythrocytes is first carried out by subjecting the latter to hypotonic conditions. The erythrocytes swell, opening the pores. The bisphosphonate is then added and penetrates inside the erythrocytes. Preferably, a solution of bisphosphonate is gradually added, and then the mixture is left to incubate for, for example, from 10 to 60 minutes, typically approximately 30 minutes. Isotonic conditions are subsequently re-established and the pores are thus resealed or re-closed, such that the erythrocytes stably encapsulate the bisphosphonate.

According to the invention, the erythrocytes are treated with a chemical agent under conditions which promote targeting of the bone marrow. This chemical agent promotes recognition by the phagocytic cells (macrophages and dendritic cells) of the bone marrow.

The chemical treatment is carried out on the erythrocytes encapsulating the bisphosphonate.

A preferred chemical agent which is compatible with clinical use in humans is bis(sulphosuccinimidyl) suberate (abbreviated to BS3 or BS³; CAS 82436-77-9). A solution of this agent is advantageously used.

A subject of the invention is also a suspension of erythrocytes, or a medicament comprising such a suspension, comprising erythrocytes encapsulating the bisphosphonate and the membrane of which has been treated in order to promote recognition by the phagocytic cells of the bone marrow, using a chemical agent, preferably BS3. The BS3 is preferably used alone, with the exclusion of any other chemical or biological agent, such as ZnCl₂. According to a first embodiment, the bisphosphonate is a first-generation bisphosphonate. According to a second embodiment, the bisphosphonate is a second-generation bisphosphonate. According to a third embodiment, the bisphosphonate is a third-generation bisphosphonate. Nonlimiting examples of bisphosphonates include: etidronate, clodronate, tiludronate, pamidronate, in particular the disodium form, alendronate, incadronate, ibandronate, neridronate, risedronate and zoledronate. According to one embodiment, the bisphosphonate is selected from: etidronate, tiludronate, pamidronate, in particular the disodium form, alendronate, incadronate, ibandronate, neridronate, risedronate and zoledronate.

According to one characteristic of the method of treatment with BS3, the suspension of erythrocytes encapsulating the bisphosphonate is brought into contact with the BS3 for a suitable period of time, which may be, in particular, between approximately 10 min and approximately 1 hour. This period of time is advantageously between approximately 15 min and approximately 45 min, preferably between approximately 20 and approximately 40 min, typically of the order of 30 min.

According to another characteristic of the method of treatment with BS3, this incubation is preferably carried out at ambient temperature, in particular between 18 and 25°C.

According to another characteristic of the method of treatment with BS3, the suspension of erythrocytes containing the bisphosphonate is pre-washed with a suitable buffer, e.g. PBS.

According to another characteristic, the suspension of erythrocytes treated with BS3 is brought to a concentration of between approximately 0.5 × 10⁶ and approximately 5 × 10⁶ cells/µl, typically between approximately 1 × 10⁶ and approximately 3 × 10⁶ cells/µl, before being brought into contact with the solution of BS3.

According to another characteristic, a solution of BS3 is used to obtain a final concentration of BS3 of between approximately 0.1 and approximately 6 mM, preferably between approximately 0.5 and approximately 3 mM, typically approximately 1 mM in the suspension. A solution of BS3 of approximately 2 mM may in particular be used. A buffered solution of BS3, preferably containing glucose and phosphate buffer, is preferably used to obtain the desired final concentration of BS3, in particular of approximately 1 mM. According to one characteristic, the buffered solution of BS3 is advantageously at a pH of between approximately 7.2 and approximately 7.6, preferably a pH of approximately 7.4. According to another characteristic, the buffered solution of BS3 has an osmolarity of between approximately 280 and approximately 320 mOsm.

The incubation can be stopped using an agent such as Tris-HCl, and then the mixture is centrifuged, and the cells are washed and resuspended in a suitable buffer; such as SAG-BSA. The mixture is left to incubate before centrifugation for a few minutes, in particular from 1 to 10 minutes, at ambient temperature.

The suspension may be ready for use and may have a haematocrit suitable for being administered without dilution.

It may also be packaged in such a way as to have to be diluted before administration.

According to the invention, the haematocrit of the ready-for-use suspension is advantageously between approximately 40% and approximately 70%, preferably between approximately 45% and approximately 55%, better still approximately 50%.

In its form for dilution, the haematocrit may be high, in particular between approximately 60% and approximately 90%.

The suspension is preferably packaged in a volume of approximately 10 to approximately 250 ml. The packaging is preferably in a blood bag of the type suitable for a blood transfusion. The encapsulated amount of bisphosphonate corresponding to the medical prescription is preferably contained entirely in the blood bag.

For example, the suspension corresponding to one dose, for example one blood bag, comprises from 1 to 40 mg of bisphosphonate, in particular from 2 to 10 mg.

According to one characteristic of the invention, the erythrocytes to be administered are in suspension in a pharmaceutically acceptable saline solution (for example, standard medium for red blood cells, in particular a solution containing NaCl and one or more ingredients selected from glucose, dextrose, adenine and mannitol; e.g. SAG-mannitol or ADsol). This solution is capable of preserving the erythrocytes, and may also include a preservation additive, such as L-carnitine.

The subject of the invention is thus a method for preventing or treating bone metastases or other bone diseases. This method comprises the administration, to the patient, of a formulation or of a medicament according to the invention.

In accordance with the invention, the administration of the formulation or medicament is carried out by intravenous or intra-arterial injection, and preferably by means of a drip from a blood bag or the like. The administration is typically carried out intravenously in the arm or via a central catheter.

From approximately 10 to approximately 250 ml of formulation (one dose) according to the invention are in particular administered. From 50 ml upwards, the use of a drip is preferred.

A treatment comprises the administration of one dose or of several doses according to the protocol decided upon. Said protocol may provide for several administrations at a monthly, two-monthly, trimestrial, semestrial or annual frequency, over the recommended period of treatment.

The techniques for encapsulating active ingredients in red blood cells are known, and the basic technique by lysis-resealing, which is preferred herein, is described in patents EP-A-101 341 and EP-A-679 101, to which those skilled in the art may refer. According to this technique, the primary compartment of a dialysis element (for example, dialysis bag or dialysis cartridge) is continuously supplied with a suspension of erythrocytes, while the second compartment contains an aqueous solution that is hypotonic with respect to the suspension of erythrocytes, in order to lyse the latter; next, in a resealing unit, the resealing of the erythrocytes is induced in the presence of the bisphosphonate, by increasing the osmotic and/or oncotic pressure, and then a suspension of erythrocytes containing the bisphosphonate is recovered. In accordance with one characteristic of the invention, it is preferred to carry out the lysis of a suspension of erythrocytes already containing the bisphosphonate to be encapsulated.

The suspension of erythrocytes encapsulating the bisphosphonate can in particular be obtained by the following method, which is also a subject of the invention:
1 - suspension of a red blood cell pellet in an isotonic solution at a haematocrit level greater than or equal to 65%, cooling between + 1 and + 8°C,
2 - lysis procedure, at a temperature constantly maintained between + 1 and + 8°C, comprising passage of the suspension of erythrocytes at a haematocrit level greater than or equal to 65% and of a hypotonic lysis solution cooled to between + 1 and + 8°C, into a dialysis bag or a dialysis cartridge (the cartridge is preferred),
3 - encapsulation procedure by addition, preferably gradual, of a solution of bisphosphonate to the lysed suspension, at a temperature maintained between + 1 and +8°C, preferably for an incubation period in parti cular from 10 to 60 min, typically of approximately 30 min, and
4 - resealing procedure carried out in the presence of a hypertonic solution, at a higher temperature, in particular between + 30 and + 42°C.

As a preferred variant, inspiration may be drawn from the method described in WO-A-2006/016247, which makes it possible to encapsulate the bisphosphonate effectively, reproducibly, safely and stably. The suspension of erythrocytes encapsulating the bisphosphonate can then be obtained by the following method, which is also a subject of the invention:
1 - suspension of a red blood cell pellet in an isotonic solution at a haematocrit level greater than or equal to 65%, cooling between + 1 and + 8°C,
2 - measurement of the osmotic fragility using a sample of erythrocytes from this same red blood cell pellet,
   it being possible for steps 1 and 2 to be carried out in any order (including in parallel),
3 - lysis procedure, in particular inside the same chamber, at a temperature constantly maintained between + 1 and + 8°C, comprising passage of the suspension of erythrocytes at a haematocrit level greater than or equal to 65% and of a hypotonic lysis solution cooled to between + 1 and + 8°C, into a di alysis bag or a dialysis cartridge (the cartridge is preferred); the lysis parameters being adjusted as a function of the osmotic fragility previously measured, and
4 - encapsulation procedure by addition, preferably gradual, of a solution of bisphosphonate to the lysed suspension, at a temperature maintained between + 1 and + 8°C, preferably for an incubation period of in particular from 10 to 60 min, typically of approximately 30 min, and
5 - resealing procedure carried out in a second chamber in the presence of a hypertonic solution, at a higher temperature, in particular between + 30 and + 42°C.

The term "internalization" is intended to mean penetration of the bisphosphonate inside the erythrocytes.

In particular, for the dialysis, the red blood cell pellet is suspended in an isotonic solution at a high haematocrit level, greater than or equal to 65%, and preferably greater than or equal to 70% and this suspension is cooled between + 1 and + 8°C, preferably between + 2 and + 6°C, typically around + 4°C. Acco rding to one particular embodiment, the haematocrit level is between 65% and 80%, preferably between 70% and 80%.

When it is measured, the osmotic fragility is advantageously measured on the erythrocytes just before the lysis step, in the presence or absence of bisphosphonate in the suspension. The erythrocytes or the suspension containing them is advantageously at a temperature close to or identical to the temperature selected for the lysis. According to one advantageous characteristic of the invention, the osmotic fragility measurement carried out is rapidly exploited, i.e. the lysis procedure is carried out shortly after the sample has been taken. Preferably, this period of time between the taking of the sample and the beginning of the lysis is less than or equal to 30 minutes, even better still less than or equal to 25 and even less than or equal to 20 minutes.

With regard to the way in which the lysis-resealing procedure is carried out, with the osmotic fragility being measured and taken into account, those skilled in the art may refer to WO-A-2006/016247 for further details. This document is incorporated herein by way of reference.

The present invention will now be described in greater detail by means of embodiments taken as nonlimiting examples.

### I - Example 1: Method for encapsulating zoledronate in murine and human red blood cells

### Ia - Material:

For the dialysis: dialysis cartridge (Gambro 280 fibres)

Assaying: the assaying of the zoledronate in the red blood cells is carried out by high performance liquid chromatography, HPLC, after preparation of the samples according to the following method. The RBCs encapsulating the zoledronate are lysed with 2.5 volumes of water, and then the zoledronate is extracted by precipitation of the proteins and membranes with 12% trichloroacetic acid.

The RBCs (before encapsulation), the final products RBC-Zol and RBC-LR treated or not treated with BS3, and also the supernatants thereof at D0 and at D1, are assayed in order to estimate the amount of zoledronate that has been encapsulated.

In order to improve the retention time of zoledronate on the C18 support, the compound tetrabutylammonium hydrogeno sulphate is used as ion-pairing agent.

| | |
|---|---|
| Instrument: | Shimadzu UFLC |
| Column: | Gemini C18 5 µ 110A 250 x 4.6 mm ID |
| Temperature: | 40°C |
| Injection volume: | 40 µl |
| UV detection: | 220 nm |
| Flow rate: | 0.7 ml/min |
| Mobile phase A: | 8 mM K2HPO4 - (1.39 g/l), 2 mM Na2HPO4 - (0.1 g/l), 7 mM tetrabutylammonium hydrogen sulphate - (2.7 g/l) |
| Mobile phase B: | Methanol |

### Ib - Method:

The red blood cells are centrifuged, and then washed three times in PBS. The haematocrit of the suspension is brought to 70% with PBS, before beginning the dialysis. The RBCs are dialysed at a flow rate of 2 ml/min against a low-osmolarity lysis buffer (contraflow at 15 ml/min). The lysed RBCs leaving the column are divided up into two equal volumes. The solution of Zometa^{®} (0.8 mg/ml of zoledronic acid) is gradually added (ten times) to one of the volumes of dialysed red blood cells, to reach a final concentration of 0.4 mg/ml.

As control, the other volume of dialysed red blood cells is diluted with one volume of PBS, added gradually. The two suspensions are incubated for 30 minutes at 4-8°C.

The red blood cells are resealed by adding a high-osmolarity solution (0.1 volume) and incubation for 30 minutes at 37°C. The resealed cells are washed three times in PBS containing glucose. The suspensions are brought to a haematocrit of 50%, either with SAG-mannitol supplemented or not supplemented with BSA (6%), or PBS containing glucose, or else are stored directly at a high haematocrit (80%) so as to constitute the final products RBC-Zol (zoledronate) and RBC-LR (lysed-resealed control without zoledronate).

### II - Example 2: chemical treatment with bis(sulphosuccinimidyl)suberate (BS³) on the red blood cells containing zoledronate

The suspension of red blood cells containing zoledronate is obtained as described in Example 1. This suspension is washed several times before being diluted to 1.7 × 10⁶ cells/µl, and then brought into contact with a 2 mM solution of BS³ containing 50 mM phosphate buffer, pH 7.4, and 0.09% glucose, so as to obtain a final concentration of BS³ of 1 mM. The red blood cells are incubated for 30 minutes at ambient temperature, and then the reaction is stopped by adding one volume of 20 mM Tris, 140 mM NaCl. After centrifugation for 5 minutes, the red blood cells are washed once with PBS containing glucose, and then once with SAG-mannitol supplemented or not supplemented with BSA (6%). The red blood cells are brought to a haematocrit of 50% in SAG-mannitol supplemented or not supplemented with BSA (6%), or PBS containing glucose, or else are stored directly at a high haematocrit (80%).

### III - Results for Examples 1 and 2:

### a) Encapsulations in human RBCs

The following tables give a review of 4 Zometa^{®} encapsulation experiments carried out with human red blood cells (RBC concentrate bag).

The cell data (Table 1) obtained on analysing the final products RBC-Zol at D1 demonstrate that the loaded RBCs maintain cell characteristics close to the bag RBCs and do not experience any particular damage.

**Table 1:**

| Cell data relating to human red blood cells (at D1) | | | | | | |
|---|---|---|---|---|---|---|
| Experiment | Treatment | Storage medium | MCV (µm³) | Extracellular haemoglobin (g/dl) | MCHC (g/dl) | Cell yield |
| 1 | - | PBS containing glucose | 87 | 0.81 | 25:5 | - |
| 2 | - | SAG-mannitol | 83 | 1.49 | 26.9 | 76.50% |
| 3 | - | PBS containing glucose | 80 | 0.69 | 28.8 | 78.00% |
| 4 | 1 mM BS3 | SAG-mannitol | 84 | 1.17 | 28.1 | 58.00% |

The corpuscular haemoglobin concentrations (MCHC) remain satisfactory, with values greater than 25 g/dl. This attests to a small loss of the intra-erythrocyte content during the method. The extracellular haemoglobin is less than 2 g/dl, the final products obtained are therefore of injectable quality. The yield for the 1 mM BS3 group generates a cell loss of 17%. However, the overall cell yields of the final products are all compatible with an industrial production (> 55%).

The dialysis process results in a decrease in the mean corpuscular volume (MCV) of the RBC-Zol red blood cells (80-88 µm³) compared with the red blood cells of the bag (97 µm³).

The data relating to the encapsulation of zoledronate are given in Table 2.

**Table 2**

| Data relating to the encapsulation of zoledronate (at D1) | | | | | | |
|---|---|---|---|---|---|---|
| Exp | Storage haematocrit 4°C | Storage medium | Dosed zoledronate (µg/ml) | Intracellular zoledronate (%) | Extracellular zoledronate (%) | Incorporation yield (%) |
| 1 | 50% | PBS containing glucose | 54.3 | 68 | 32 | 23 |
| 2 | 50% | SAG-mannitol | 66.2 | 83 | 17 | 33.6 |
| 3 | 80% | PBS containing glucose | 69.5 | 83 | 17 | 35.5 |
| 4 | 50% | SAG-mannitol | 56.1 | 71 | 29 | 24.5 |

Zoledronate has a critical size for encapsulation in RBCs and the most important criterion to be observed is the amount of the compound encapsulated in the RBCs at D1 compared with the extracellular proportion. Three experiments were carried out without particular treatment of the erythrocyte membranes, and the results show a satisfactory encapsulation of zoledronate at D1 (from 68% to 83% of the zoledronate in the final product is encapsulated in the RBCs). With the BS3 treatment, the encapsulation is 71%.

Table 3 shows data relating to the stability of the human red blood cells containing zoledronate. On the day of production (D0) and on the following day (D1), the extracellular haemoglobin, the haematocrit of the suspensions and the amounts of intracellular and extracellular zoledronate are measured.

| Experiment | Medium | Extracellular zol | Extracellular zol | Extra-cellular Hb | Extracellular Hb | Haematocrit | | Haematocrit yield |
|---|---|---|---|---|---|---|---|---|
| | | | | | | D0(%) | | |
| | | D0(%) | D1(%) | D0 (g/dl) | D1 (g/dl) | D1(%) | | (%) |
| 1 | PBS containing glucose | 13 | 32 | 0.30 | 0.81 | 49.4 | 47.8 | 96.76% |
| 2 | SAG-mannitol | 13 | 17 | 0.45 | 1.49 | 50.0 | 48.4 | 96.80% |
| 4 | SAG-mannitol | 16 | 29 | 0.26 | 1.17 | 51.1 | 49.5 | 96.87% |

An increase in extracellular zoledronate between D0 and D1 is observed. This can be correlated to the extracellular haemoglobin which increases between D0 and D1, and also to the cell loss (haematocrit yield). The release of zoledronate into the extracellular medium therefore comes essentially from the rupturing of red blood cells which had not resealed properly after the dialysis, and not from passive or active leaking through the erythrocyte membrane. The results show good storage under the various conditions.

### b) Encapsulations in murine RBCs

The following table gives a review of 3 Zométa^{®} encapsulation experiments carried out with murine red blood cells (whole blood). Just as for the human RBCs, the RBC-Zol maintain cell characteristics close to the whole blood.

**Table 4**

| Cell data relating to murine red blood cells (at D1) | | | | | |
|---|---|---|---|---|---|
| Experiment | Treatment | MCV (µm³) | Extracellular haemoglobin (g/dl) | Osmotic fragility (g/l) | MCHC (g/dl) |
| 5 | 1 mM BS3 | 43 | 1.99 | 1.67 | 32.2 |
| 6 | - | 46 | 3.38 | 1.47 | 26.7 |
| 7 | 1 mM BS3 | 43 | 2 | - | 31.5 |

The corpuscular haemoglobin concentrations (MCHC) are very satisfactory. The extracellular haemoglobin is higher than in the case of the human RBCs, since the mouse red blood cells are more fragile. The dialysis process results in a mean corpuscular volume of the RBC-Zol red blood cells (43-46 µm³) which is homogeneous from one experiment to another.

Table 5 shows data relating to the encapsulation of zoledronate in murine RBCs (measurement at D1). The final suspension is a concentrated suspension with a haematocrit of 80%.

**Table 5**

| Data relating to the encapsulation of zoledronate (murine RBCs) (D1) | | | | | |
|---|---|---|---|---|---|
| Experiment | Treatment | Dosed zoledronate (µg/ml) | Intracellular zoledronate (%) | Extracellular zoledronate (%) | Incorporation yield (%) |
| 11 | 1 mM BS3 | 55.7 | 92 | 8 | 28 |
| 12 | - | 79.8 | 70 | 30 | 30 |
| 13 | 1 mM BS3 | 55.1 | 78 | 22 | 24 |

The results show a satisfactory encapsulation at D1.

### V - Example 3: Validation of the targeting of the bone marrow with the BS³ treatment:

The fluorochrome FITC-dextran (70 kDa) was encapsulated in murine red blood cells (OF1 mice) by the method of hypotonic dialysis in a column. The blood is precentrifuged, and then washed three times in PBS. The haematocrit is brought to 70% in the presence of FITC-dextran, added at a final concentration of 8 mg/ml, before starting the dialysis. The RBCs are dialysed at a flow rate of 2 ml/min against a low-osmolarity lysis buffer (contraflow at 15 ml/min). The lysed RBCs leaving the column are resealed by adding a high-osmolarity solution and incubating for 30 minutes at 37°C. After two washes in PBS containing glucose, the RBCs are diluted to 1.7 × 10⁶ cells/µl, before being brought into contact with a 10 mM solution of BS³ containing 50 mM phosphate buffer, pH 7.4, and 0.09% glucose. The RBCs are incubated for 30 minutes at ambient temperature, and the reaction is then stopped by adding one volume of 20 mM Tris, 140 mM NaCl. After centrifugation for 5 minutes, the RBCs are washed once with PBS containing glucose, and then once with SAG-mannitol supplemented with BSA (6%). The red blood cells are brought to a haematocrit of 50% so as to constitute the final product, which is injected into the mouse at D1. The mouse is sacrificed 1 h 30 after injection, and then the bone marrow isolated from the femurs is placed in Tissue-Tek for freezing in nitrogen. 10 µm cryostat sections are cut for immunohistochemical analysis. After fixing in acetone, double labelling is carried out, demonstrating the FITC (DAB, brown) and the F4/80 macrophages (new fuschin, red).

Observation of the sections under a microscope shows colocalization of the macrophages and of the dextran. The observation confirms the incorporation of the dextran by the macrophages via phagocytosis of the red blood cells.

The flow cytometry analysis (FC500 Beckman Coulter) gives the following information.

**Table 6: percentage of fluorescent cells in the bone marrow 1 h 30 after intravenous injection of the RBCs into the mice**

| Treatments | Number of total fluorescent cells |
|---|---|
| RBC-Dextran | 5.3% |
| RBC-Dextran-BS3 | 7.4% |

The flow cytometry analysis shows that, 1 h 30 after injection, approximately 7% and 5% of the cells of the bone marrow are fluorescent in the case of the BS3 treatment and in the untreated case.

Table 7 shows the percentage of phagocytic cells which have phagocytized treated or untreated red blood cells.

| Treatments | F4/80 macrophages | Dendritic cells |
|---|---|---|
| RBC-Dextran | 5.2% | 7.9% |
| RBC-Dextran-BS3 | 9.5% | 16.3% |

The BS3 treatment induces a more rapid and greater uptake, by phagocytosis, of the RBCs in the bone marrow than in the absence of treatment.

### Conclusions

▪ Zoledronate can be stably encapsulated in RBCs, which may or may not have been membrane-treated with BS3.
▪ The amounts encapsulated are largely compatible with the amounts used clinically. The encapsulation experiments (with 1 mM BS3 treatment) showed that final products containing 56.1 µ/ml of zoledronate were obtained. It would therefore be necessary to give a person an infusion of approximately 71 ml of RBC-Zol in order to have the equivalent of 4 mg.
▪ The treatment with 1 mM BS3 makes it possible to target the cells of the bone marrow.

All this validates the use of RBCs as a carrier of zoledronate for targeting the bone marrow and in the context of bone metastases or other bone diseases.

## Claims

1. Suspension of erythrocytes encapsulating a second-generation or third-generation bisphosphonate, in which the erythrocytes encapsulating the bisphosphonate have undergone a chemical treatment with a chemical agent so as to promote targeting of the bone marrow, for use as a medicament for the prevention and treatment of bone metastases.

2. Suspension of erythrocytes according to Claim 1, in which the chemical treatment is carried out with a solution of bis(sulphosuccinimidyl)suberate (BS3).

3. Suspension of erythrocytes according to Claim 2, in which the suspension of erythrocytes encapsulating the bisphosphonate is brought into contact with the BS3 for a period of time of between 10 min and 1 hour, in particular between 15 min and 45 min, preferably between 20 and 40 min, typically of the order of 30 min.

4. Suspension of erythrocytes according to Claim 2 or 3, in which the incubation with the BS3 is at ambient temperature.

5. Suspension of erythrocytes according to one of Claims 2 to 4, in which, before incubation with the BS3, the suspension of erythrocytes containing the bisphosphonate is washed with a suitable buffer, preferably PBS.

6. Suspension of erythrocytes according to one of Claims 2 to 5, in which the suspension of erythrocytes encapsulating the bisphosphonate is brought to a concentration of between 0.5 × 10⁶ and 5 × 10⁶ cells/µl, preferably between 1 × 10⁶ and 3 × 10⁶ cells/µl, before being brought into contact with the solution of BS3.

7. Suspension of erythrocytes according to one of Claims 2 to 6, in which a solution of BS3 is used to obtain a final concentration of BS3 of between 0.1 and 6 mM, preferably between 0.5 and 3 mM, even better still of approximately 1 mM in the suspension.

8. Suspension of erythrocytes according to one of Claims 2 to 7, in which a buffered solution of BS3 having an osmolarity of between 280 and 320 mOsm and a pH of between 7.2 and 7.6, preferably of 7.4, is used.

9. Suspension of erythrocytes according to Claim 8, in which the solution of BS3 comprises glucose and phosphate buffer.

10. Suspension of erythrocytes according to any one of claims 1 to 9, in which the bisphosphonate is selected from: pamidronate, in particular the disodium form, alendronate, incadronate, ibandronate, neridronate, risedronate and zoledronate.

11. Suspension of erythrocytes according to Claim 10, in which the bisphosphonate is zoledronate.

## Patentansprüche

1. Suspension von roten Blutkörperchen, in denen ein Bisphosphonat der zweiten Generation oder der dritten Generation eingekapselt ist, wobei die roten Blutkörperchen, in denen das Bisphosphonat eingekapselt ist, einer chemischen Behandlung mit einer chemischen Substanz unterzogen worden sind, um Knochenmarktargeting zu fördern, zur Verwendung als ein Medikament zur Prävention und Behandlung von Knochenmetastasen.

2. Suspension von roten Blutkörperchen gemäß Anspruch 1, wobei die chemische Behandlung mit einer Lösung von Bis(sulphosuccinimidyl)suberat (BS3) erfolgt.

3. Suspension von roten Blutkörperchen gemäß Anspruch 2, wobei die Suspension von roten Blutkörperchen, in denen das Bisphosphonat eingekapselt ist, für eine Dauer zwischen 10 min und 1 Stunde, insbesondere zwischen 15 min und 45 min, bevorzugt zwischen 20 und 40 min, üblicherweise in der Größenordnung von 30 min, in Kontakt mit dem BS3 gebracht wird.

4. Suspension von roten Blutkörperchen gemäß Anspruch 2 oder 3, wobei die Inkubation mit dem BS3 unter Umgebungstemperatur erfolgt.

5. Suspension von roten Blutkörperchen gemäß einem der Ansprüche 2 bis 4, wobei vor Inkubation mit dem BS3 die Suspension von roten Blutkörperchen, in denen das Bisphosphonat enthalten ist, mit einem geeigneten Puffer, bevorzugt PBS, gewaschen wird.

6. Suspension von roten Blutkörperchen gemäß einem der Ansprüche 2 bis 5, wobei die Suspension von roten Blutkörperchen, in denen das Bisphosphonat eingekapselt ist, vor in Kontakt bringen mit der Lösung von BS3 auf eine Konzentration zwischen 0,5 x 10⁶ und 5 x 10⁶ Zellen/µl, bevorzugt zwischen 1 x 10⁶ und 3 x 10⁶ Zellen/µl, gebracht wird.

7. Suspension von roten Blutkörperchen gemäß einem der Ansprüche 2 bis 6, wobei eine Lösung von BS3 verwendet wird, um eine Endkonzentration an BS3 in der Suspension zwischen 0,1 und 6 mM, bevorzugt zwischen 0,5 und 3 mM, besser sogar noch von ungefähr 1 mM, zu erhalten.

8. Suspension von roten Blutkörperchen gemäß einem der Ansprüche 2 bis 7, wobei eine gepufferte Lösung von BS3 mit einer Osmolarität zwischen 280 und 320 mOsm und einem pH-Wert zwischen 7,2 und 7,6, bevorzugt von 7,4, verwendet wird.

9. Suspension von roten Blutkörperchen gemäß Anspruch 8, wobei die Lösung von BS3 Glucose und Phosphatpuffer enthält.

10. Suspension von roten Blutkörperchen gemäß einem der Ansprüche 1 bis 9, wobei das Bisphosphonat ausgewählt ist aus: Pamidronat, insbesondere der Dinatriumform, Alendronat, Incadronat, Ibandronat, Neridronat, Risedronat und Zoledronat.

11. Suspension von roten Blutkörperchen gemäß Anspruch 10, wobei das Bisphosphonat Zoledronat ist.

## Revendications

1. Suspension d'érythrocytes encapsulant un bisphosphonate de deuxième ou troisième génération, dans laquelle les érythrocytes encapsulant le bisphosphonate ont subi un traitement chimique avec un agent chimique de manière à favoriser le ciblage de la moelle osseuse, pour utilisation comme médicament pour la prévention et le traitement des métastases osseuses.

2. Suspension d'érythrocytes selon la revendication 1, dans laquelle le traitement chimique est réalisé à l'aide d'une solution de Bis(sulfosuccinimidyl) suberate (BS3).

3. Suspension d'érythrocytes selon la revendication 2, dans laquelle la suspension d'érythrocytes encapsulant le bisphosphonate est mise en contact avec le BS3 pendant une durée comprise entre 10 min et 1 heure, notamment entre 15 min et 45 min, de préférence entre 20 et 40 min, typiquement de l'ordre de 30 min.

4. Suspension d'érythrocytes selon la revendication 2 ou 3, dans laquelle l'incubation avec le BS3 est à température ambiante.

5. Suspension d'érythrocytes selon l'une des revendications 2 à 4, dans laquelle, avant incubation avec le BS3, la suspension d'érythrocytes renfermant le bisphosphonate est lavée à l'aide d'un tampon approprié, de préférence du PBS.

6. Suspension d'érythrocytes selon l'une des revendications 2 à 5, dans laquelle la suspension d'érythrocytes encapsulant le bisphosphonate est amenée à une concentration comprise 0,5 10⁶ et 5 10⁶ cellules/µl, de préférence entre 1 10⁶ et 3 10⁶ cellules/µl, avant d'être mise en contact avec la solution de BS3.

7. Suspension d'érythrocytes selon l'une des revendications 2 à 6, dans laquelle on emploie une solution de BS3 pour obtenir une concentration finale en BS3 comprise entre 0,1 et 6 mM, de préférence entre 0,5 et 3 mM, mieux encore d'environ 1 mM dans la suspension.

8. Suspension d'érythrocytes selon l'une des revendications 2 à 7, dans laquelle on emploie une solution tamponnée de BS3 ayant une osmolarité comprise entre 280 et 320 mOsm et un pH compris entre 7,2 et 7,6, de préférence de 7,4.

9. Suspension d'érythrocytes selon la revendication 8, dans laquelle, la solution de BS3 comprend du glucose et du tampon phosphate.

10. Suspension d'érythrocytes selon l'une quelconque des revendications 1 à 9, dans laquelle le bisphosphonate est choisi parmi : pamidronate, notamment disodique, alendronate, incadronate, ibandronate, neridronate, risedronate et zolédronate.

11. Suspension d'érythrocytes selon la revendication 10, dans laquelle le bisphosphonate est le zolédronate.
